# EUROPEAN PATENT SPECIFICATION

(11) **EP 2 237 810 B1**
(45) Date of publication and mention of the grant of the patent: **01.05.2013**
(21) Application number: 08869926.9
(22) Date of filing: 31.12.2008
(51) Int. Cl.: A61L 31/06, A61L 31/14

(54) **BIODEGRADABLE POLYMERS**
BIOLOGISCH ABBAUBARE POLYMERE
POLYMÈRES BIODÉGRADABLES

(30) Priority: 31.12.2007 US 967838
(43) Date of publication of application: 13.10.2010
(73) Proprietor: Boston Scientific Scimed, Inc., Maple Grove, MN 55311-1566 (US)
(72) Inventor: BENCO, John, Holliston MA 01746 (US); BODEN, Mark, Harrisville RI 02830 (US)
(74) Representative: Vossius & Partner
(86) International application number: PCT/US2008/088614
(87) International publication number: WO 2009/088912

(56) References cited:
- WO-A1-2005/056608
- US-A1- 2006 222 681
- US-A1- 2007 155 926

## Description

### FIELD OF THE INVENTION

The present invention relates generally to implantable or insertable medical devices that contain biodegradable polymers having internal disulfide linkages and a therapeutic agent that is covalently linked to the polymer by a disulfide bond.

### BACKGROUND OF THE INVENTION

Biodegradable polymers have a wide range of uses, including uses in medical device applications and; in particular, in implantable or insertable medical devices. In such cases, the device may be coated with a biodegradable polymer, for example, to initiate, enhance or improve initial biocompatibility upon implantation. Once the device is implanted and stabilizes within the body, it is desirable to have the polymer degrade in many cases. There are many such polymers which have been used in medical devices, predominantly from the polyester family, including polylactides and polyglycolides and poly(lactides-co-glycolides). However, a significant issue is that, when these polymers degrade, the degradation products possess a wide range of molecular weights and therefore represent an unpredictable system, leading to a range of biological effects, including, for example, foreign body responses from crystalline particulates, inflammatory responses due to the presence of acidic decomposition by-products and/or dose dumping as mechanical integrity is lost. To overcome this major limitation a new biodegradable polymeric system is desired. Ideally, such a system would yield well-controlled molecular weight degradation products, would possess the ability to incorporate groups which can be adjusted to tune the rate of biodegradation, and would possess facile synthetic attributes, among other characteristics.

US 2006/0222681 A1 discloses polymeric release regions which contain one or more partially biodegradable progenitor polymers composed of a plurality of biostable polymer portions linked via one or more biodegradable linkages. US 2007/0155926 A1 discloses polymers comprising a polymer backbone comprising one or more degradable units. The polymers can be degraded by hydrolysis, photolysis or by biodegradation in an external environment or within a living body. WO 2005/056608 A1 describes compounds, such as macromolecules that have been modified to facilitate crosslinking by introduction of at least one hydrazide-reactive group and/or aminooxy-reactive group and which can be used for delivering bioactive agents.

### SUMMARY OF THE INVENTION

According to the present invention, implantable or insertable medical devices as defined in the annexed claims are provided that contain biodegradable polymeric regions which in turn contain polymers that have a plurality of well defined polymer segments linked by disulfide linkages.

An advantage of such biodegradable polymers is that they are ultimately broken down into degradation products of very uniform size.

These and other aspects, embodiments and advantages of the present invention will become immediately apparent to those of ordinary skill in the art upon review of the Detailed Description and Claims to follow.

### DETAILED DESCRIPTION OF THE INVENTION

A more complete understanding of the present invention is available by reference to the following detailed description of numerous aspects and embodiments of the invention. The detailed description of the invention which follows is intended to illustrate but not limit the invention.

As noted above, the present invention makes use of polymers that have a plurality of polymer segments linked by disulfide linkages. When these linkages are broken down a plurality of smaller polymers of lower molecular weight (also referred to herein as "polymer degradation products" or "polymer fragments") are produced.

According to the present invention, implantable or insertable medical device as defined in the annexed claims are provided that contain biodegradable polymeric regions which in turn contain polymers that have a plurality of polymer segments linked by disulfide linkages.

Disulfide linkages may be broken down, for example, by exposure to reducing agents, nucleophiles, electrophiles.photochemically, or by enzymatic reduction. Disulfide linkages are known to biodegrade (see, e.g., X.Z. Shu et al Biomaterials, 2003, 24, 3825-3834), but have received limited attention, apparently due to synthetic obstacles as well as lack of control over the rate of incorporation.

As discussed in more detail below, disulfide linkages can be incorporated into a wide range of polymeric materials. For example, they can be incorporated into hydrophobic polymers and thus allow for a biodegradation of polymeric systems which are typically non-degradable in vivo. Conversely, the linkages can be incorporated into hydrophilic polymers, if desired.

Medical devices for the practice of the present invention are implantable or insertable medical devices, for example, stents (including coronary vascular stents, peripheral vascular stents, cerebral, urethral, ureteral, biliary, tracheal, gastrointestinal and esophageal stents), stent coverings, catheters (*e.g*., renal or vascular catheters such as balloon catheters and various central venous catheters), guide wires, balloons, filters (*e.g*., vena cava filters and mesh filters for distil protection devices), stent grafts, vascular grafts, abdominal aortic aneurysm (AAA) devices (e.g., AAA stents, AAA grafts, etc.), vascular access ports, dialysis ports, embolization devices including cerebral aneurysm filler coils (including Guglilmi detachable coils and metal coils), embolic agents, bulking agents, septal defect closure devices, myocardial plugs, patches, pacemakers, lead coatings including coatings for pacemaker leads, defibrillation leads and coils, ventricular assist devices including left ventricular assist hearts and pumps, total artificial hearts, shunts, valves including heart valves and vascular valves , anastomosis clips and rings, cochlear implants, tissue bulking devices, and tissue engineering scaffolds for cartilage, bone, skin and other in vivo tissue regeneration, sutures, suture anchors, tissue staples and ligating clips at surgical sites, cannulae, metal wire ligatures, urethral slings, hernia "meshes", artificial ligaments, orthopedic prosthesis such as bone grafts, bone plates, fins and fusion devices, joint prostheses, spinal discs and nuclei, as well as any coated substrate that is implanted or inserted into the body.

In some embodiments, the biodegradable polymeric regions of the present invention correspond to an entire medical device. In other embodiments, the biodegradable polymeric regions correspond to one or more portions of a medical device. For instance, the biodegradable polymeric regions can be in the form of one or more medical device components, in the form of one or more fibers which are incorporated into a medical device, in the form of one or more polymeric layers formed over all or only a portion of an underlying substrate, and so forth. Materials for use as underlying medical device substrates include ceramic, metallic and polymeric substrates. The substrate material can also be a carbon- or silicon-based material, among others. Layers can be provided over an underlying substrate at a variety of locations and in a variety of shapes (e.g., in the form of a series of rectangles, stripes, or any other continuous or noncontinuous pattern). As used herein a "layer" of a given material is a region of that material whose thickness is small compared to both its length and width (e.g., 20% or less, frequently much less). As used herein a layer need not be planar, for example, taking on the contours of an underlying substrate. Layers can be discontinuous (e.g., patterned).

As used herein, a "polymeric region" is a region (e.g., an entire device, a device component, a device coating layer, etc.) that contains polymers, for example, from 50 wt% or less to 75 wt% to 90 wt% to 95 wt% to 97.5 wt% to 99 wt% or more polymers.

As used herein, "polymers" are molecules containing multiple copies (e.g., from 2 to 5 to 10 to 25 to 50 to 100 to 250 to 500 to 1000 or more copies) of one or more constitutional units, commonly referred to as monomers. As used herein, the term "monomers" may refer to the free monomers and those that are incorporated into polymers, with the distinction being clear from the context in which the term is used.

Polymers may take on a number of configurations, which may be selected, for example, from cyclic, linear and branched configurations, among others. Branched configurations include star-shaped configurations (e.g., configurations in which three or more chains emanate from a single branch point), comb configurations (e.g., configurations having a main chain and a plurality of side chains, also referred to as "graft" configurations), dendritic configurations (e.g., arborescent and hyperbranched polymers), network configurations (e.g., crosslinked polymers) and so forth.

As used herein, "homopolymers" are polymers that contain multiple copies of a single constitutional unit (monomer). "Copolymers" are polymers that contain multiple copies of at least two dissimilar constitutional units (monomers), examples of which include random, statistical, gradient, periodic (e.g., alternating) and block copolymers.

As used herein, a "segment" is a portion of a polymer.

As used herein, a "chain" is a linear polymer or a portion thereof, for example, a linear polymer segment.

As used herein, a "biodegradable" polymeric region is a region which contains polymers that are broken down in vivo into a plurality of smaller polymers of lower molecular weight.

The biodegradable polymeric region is a non-crosslinked biodegradable polymeric region. Non-crosslinked regions are advantageous, for example, in that they are generally easier to process into various forms than are crosslinked regions (e.g., they may be sprayed, extruded, etc.).

As used herein, a "non-crosslinked polymeric region" is a region in which the polymer molecules forming the region, whether linear, branched, etc., are not covalently bound to one another such that they form a single high molecular weight network Non-crosslinked polymeric regions, however, may contain individual polymer molecules having one or more cross-linked portions (i.e., molecules may be cross-linked but not the polymeric region itself). For example, polymer molecules with cross-linked cores and linear arms are described further below (e.g., a star copolymer with a crosslinked polydimethacrylate core and polymethylmethacrylate arms, 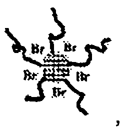 among others) which may be employed in non-crosslinked polymeric regions of the invention.

As used herein, "polydispersity" is the ratio of weight average molecular weight to number average molecular weight. It gives an indication of the molecular weight distribution of a polymer sample, with values of 1.0 to 1.5 representing a narrow molecular weight distribution. The polydispersity has a value of one when all polymers within a sample are the same size.

As noted above, the present invention provides implantable or insertable medical devices that contain biodegradable polymeric regions which in turn contains polymers that have a plurality of polymer segments linked by disulfide linkages.

An advantage of such devices is that the molecular weight distribution of the degradation products can be tuned, as well as the rate of degradation. As such, the range of biological effects (e.g. inflammatory responses, etc.) can be controlled. For example, in some embodiments, the polymer degradation products that arise upon degradation of the disulfide bonds within these polymers have a narrow polydispersity, for example, one ranging from 1.1 to 1.2 to 1.3 to 1.4 to 1.5.

Another advantage is that, the devices can be used as a vehicle for the delivery of therapeutic agents. Due to the tunable nature of the system, the kinetics of therapeutic agent release can be tailored to the desired application.

As a general rule, the solubility of a given synthetic polymer in a given liquid (e.g., a physiological fluid) will increase with a decrease in molecular weight. Moreover, for many polymers, there is a critical molecular weight below which the polymer can be dissolved. This varies, of course, with the degree of hydrophilicity of the polymer in question, among other factors. In various embodiments of the invention, the size of the original polymer is above this critical molecular weight (and therefore insoluble) whereas the size of the polymer degradation products is selected to be below this critical molecular weight (and therefore soluble). Moreover, in devices where the polymer fragments are cleared from the body via the kidneys, the molecular weight of the polymer degradation products, even if soluble, should not be so large as to inhibit removal of the fragments from the body via this pathway. In this regard, in certain embodiments, the polymer fragments may not exceed 30 kDa, more preferably may not exceed 20 kDa, and even more preferably may not exceed 10 kDa in molecular weight.

In certain embodiments, the polymeric regions of the devices of the invention are further provided with a reducing agent, for example, thiopropyl-agarose, 2-mercaptoethanol, dithiothreitol, tri-n-butyl phosphine, tris-2-carboxyethyl phosphine, glutathione, or another chain lysis catalyst whose release can be triggered at a therapeutically opportune time to accelerate degradation of the polymeric regions. For example, a reducing agent may be encapsulated within a microcapsule whose walls contain magnetic or metallic particles, and an external alternating magnetic field can be used to induce motion (e.g., vibration, rotation) or heat (e.g., via eddy currents) in the particles, thereby breaking the capsule shell or increasing its permeability. As a specific example of such an encapsulated system, see, e.g., Z. Lu et al., Langmuir, 21 (5), 2042 - 2050, 2005, in which a magnetic field is used to modulate the permeability of polyelectrolyte microcapsules, which are prepared by layer-by-layer self-assembly and which contain ferromagnetic gold-coated cobalt nanoparticles embedded inside the capsule walls. An external alternating magnetic field is applied to rotate the embedded nanoparticles, which disturbs and distorts the capsule wall and drastically increases its permeability to macromolecules, specifically, FITC-labeled dextran.

Biodegradable polymers for use in the present invention may be formed using "living" free radical polymerization processes such as metal-catalyzed atom transfer radical polymerization (ATRP) or another radical polymerization process. Living free radical polymerizations, also called controlled free radical polymerizations (CRP), are preferred in various embodiments of the invention, because they combine the undemanding nature of free radical polymerization with the power to control polydispersities, architectures, and molecular weights that living polymerization processes provide. CRP methods are well-detailed in the literature and are described, for example, in an article by Pyun and Matyjaszewski, "Synthesis of Nanocomposite Organic/Inorganic Hybrid Materials Using Controlled/"Living" Radical Polymerization," Chem. Mater., 13:3436-3448 (2001); B. Reeves, "Recent Advances in Living Free Radical Polymerization," November 20, 2001, University of Florida; and T. Kowalewski et al., "Complex nanostructured materials from segmented copolymers prepared by ATRP," Eur. Phys. J. E, 10, 5-16 (2003).

ATRP is a particularly appealing free radical polymerization technique, as it is tolerant of a variety of functional groups (*e.g*., alcohol, amine, and sulfonate groups, among others) and thus allows for the polymerization of many monomers. In monomer polymerization via ATRP, radicals are commonly generated using organic halide initiators and transition-metal complexes. Some typical examples of organic halide initiators include alkyl halides, haloesters (e.g., methyl 2-bromopropionate, ethyl 2-bromoisobutyrate, etc.) and benzyl halides (e.g., 1-phenylethyl bromide, benzyl bromide, etc.). A wide range of transition-metal complexes may be employed, including a variety of Ru-, Cu-, Os- and Fe-based systems. Examples of monomers that may be used in ATRP polymerization reactions include various unsaturated monomers such as alkyl acrylates, alkyl methacrylates, hydroxyalkyl methacrylates, vinyl esters, vinyl aromatic monomers, acrylamide, methacrylamide, acrylonitrile, and 4-vinylpyridine, among others. In ATRP, at the end of the polymerization, the polymer chains are capped with a halogen atom that can be readily transformed via S_{N}1, S_{N}2 or radical chemistry to provide other functional groups such as amino groups, among many others. Functionality can also be introduced into the polymer by other methods, for example, by employing initiators that contain functional groups which do not participate in the radical polymerization process. Examples include initiators with epoxide, azido, amino, hydroxyl, cyano, and allyl groups, among others. In addition, functional groups may be present on the monomers themselves.

It has further been found that CRP processes such as ATRP are uniquely suited for the creation of biodegradable polymeric systems that are based on disulfide linkages. Moreover, as noted above, ATRP generated polymers exhibit well controlled polydispersities, typically with a polydispersity of less than 1.5 as well as linear molecular weight to conversion profiles allowing for predicable molecular weights.

As a result, polymers can be created with disulfide linkages that, upon degradation, yield products of known size and composition. This fact, for example, allows for the tuning of degradation products and rates, both of which are highly desirable for medical device applications.

Polymers with internal disulfide linkages have been synthesized. For example, N.V. Tsarevsky et al., Macromolecules 2005, 38, 3087-3092, describe the formation of degradable polymethacrylates with internal disulfide linkages 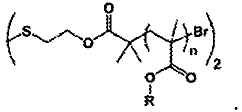 More particularly, polymers of methyl methacrylate, *tert*-butyl methacrylate and benzyl methacrylate are formed using a difunctional dihalide initiator with an internal disulfide linkage, specifically, bis[2-(2-bromoisobutyryloxy)ethyl] disulfide, 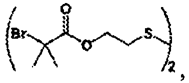 with a suitable ATRP catalyst. The disulfide bond was cleaved to thiols by reduction with tributylphosphine.

Similarly, N. V. Tsarevsky et al., Macromolecules 2002, 35, 9009-9014, describe the formation of degradable polystyrenes with internal disulfide linkages, 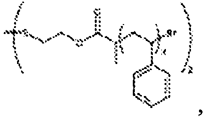 by ATRP under similar conditions using a similar dihalide initiator with an internal disulfide linkage, specifically, the 2-bromopropionic acid diester of bis(2-hydroxyethyl) disulfide, 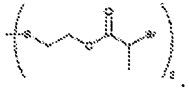 The internal disulfide bond was cleaved by reduction with dithiothreitol to yield corresponding thiol-terminated polystyrenes of approximately half the molecular weight of the parent compound.

Also described is the synthesis of dibromo-terminated polystyrene, 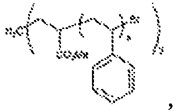 under similar ATRP conditions using dimethyl 2,6-dibromoheptanedioate as the initiator. Because the resulting arms are halide-terminated, further chain extension with additional monomer may be conducted. Thiodimethylformamide was employed to convert the bromine end groups to thiol functionalities, 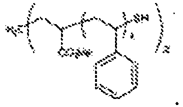 Because the obtained polymers are difunctional, they can be coupled with one another into higher molecular weight polymers with multiple internal disulfide bridges, 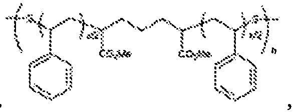 for example, via oxidation with FeCl₃.

The above polymers are linear polymers. Further tuning of the biodegradation process (and thus the therapeutic agent release in some embodiments), can be accomplished through the creation of more complex structures. Examples include are star polymers and so called miktoarm stars (i.e., star polymers with chemically different arms). The ability to create star polymers also leads to the possibility of creating hyperbranched or dendrimer type systems.

As a specific example, in H. Gao et al., Macromolecules, 2005, 38, 5995-6004, a proposed synthesis of degradable stars polymer via ATRP is described in which a dimethacrylate compound with a degradable disulfide linkage, 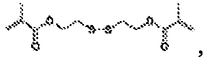 is polymerized in the presence of a linear macroinitiator, 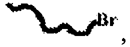 specifically, bromo-terminated polymethylmethacrylate, to form a star copolymer with a crosslinked polydimethacrylate core and polymethylmethacrylate arms, 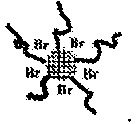 Due to the presence of the residual bromine groups in the core, synthesis of additional polymer chains, e.g., polybutylacrylate chains, can proceed via ATRP from the core, which acts as a so-called super-initiator to form a miktoarm star polymer, 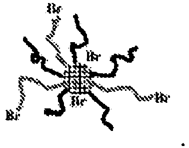 Because the resulting arms are halide-terminated, further chain extension with additional monomer may be conducted.

As noted above, CRP techniques including ATRP are very versatile, able to polymerize various unsaturated monomers including alkyl acrylates, alkyl methacrylates, hydroxyalkyl methacrylates, vinyl aromatic monomers, and vinyl esters, among others. A few specific monomers follow, along with the published glass transition temperature (Tg) of homopolymers formed therefrom.

Specific examples of alkyl acrylates include low Tg methyl acrylate (Tg 10°C), ethyl acrylate (Tg -24°C), propyl acrylate, isopropyl acrylate (Tg -11°C, isotactic), butyl acrylate (Tg -54°C), sec-butyl acrylate (Tg -26°C), isobutyl acrylate (Tg -24°C), cyclohexyl acrylate (Tg 19°C), 2-ethylhexyl acrylate (Tg -50°C), dodecyl acrylate (Tg - 3°C) and hexadecyl acrylate (Tg 35°C) and high Tg tert-butyl acrylate (Tg 43-107°C), among others.

Specific examples of alkyl methacrylates include low Tg monomers such as butyl methacrylate (Tg 20°C), hexyl methacrylate (Tg -5°C), 2-ethylhexyl methacrylate (Tg - 10°C), octyl methacrylate (Tg -20°C), dodecyl methacrylate (Tg -65°C), hexadecyl methacrylate (Tg 15°C) and octadecyl methacrylate (Tg -100°C) and high Tg monomers such as methyl methacrylate (Tg 105-120°C), ethyl methacrylate (Tg 65°C), isopropyl methacrylate (Tg 81°C), isobutyl methacrylate (Tg 53°C), t-butyl methacrylate (Tg 118°C) and cyclohexyl methacrylate (Tg 92°C), among others.

Specific examples of hydroxyalkyl (meth)acrylates, where "(meth)acrylate" is the generic term for methacrylates and acrylates, include 2-hydroxyethyl acrylate, 2-hydroxyethyl methacrylate (Tg 57°C), and 2-hydroxypropyl methacrylate (Tg 76°C), among others.

Specific examples of other (meth)acrylates include glycidyl acrylate, which has pendant oxirane rings which can be opened and used for subsequent side chain modification, tetrahydropyranyl methacrylate, trimethylsilyl methacrylate, 2-(dimethylamino)ethyl acrylate, vinyl acrylate, allyl acrylate, and benzyl acrylate, among others.

Specific examples of vinyl aromatic monomers include styrene (Tg 100°C) and 2-vinyl naphthalene (Tg 151°C) as well as alkyl substituted vinyl aromatics such as 3-methylstyrene (Tg 97°C), 4-methylstyrene (Tg 97°C) and 4-tert-butylstyrene (Tg 127°C), halo substituted vinyl aromatics such as 4-chlorostyrene (Tg 110°C), 4-bromostyrene (Tg 118°C), 4-fluorostyrene (Tg 95°C), 3-trifluoromethylstyrene and 3-4-trifluoromethylstyrene, and ester-substituted vinyl aromatics such as 4-acetoxystyrene (Tg 116°C), among others.

Specific examples of vinyl ester monomers include vinyl acetate (Tg 30°C), vinyl propionate (Tg 10 °C), vinyl benzoate (Tg 71°C), vinyl 4-tert-butyl benzoate (Tg 101°C) and vinyl cyclohexanoate (Tg 76°C), among others.

As used herein, a "low Tg polymer" is one that displays a Tg that is below body temperature, more typically from 35°C to 20°C to 0°C to -25°C to -50°C or below, and is typically soft and rubbery at body temperature. Conversely, as used herein, a "high Tg polymer block" is one that displays a Tg that is above body temperature, more typically from 40°C to 50°C to 75°C to 100°C or above, and is typically hard at body temperature. Tg can be measured by differential scanning calorimetry (DSC).

Many of the above monomers are relatively non-polar (e.g., styrene, etc.). Others are relatively polar (e.g., 2-hydroxyethyl methacrylate, etc.).

Still other monomers can be converted into monomers of differing polarity. For example, as noted above, *tert*-butyl- and benzyl-substituted methacrylates have been polymerized by ATRP. See, e.g., N.V. Tsarevsky et al Macromolecules, 2005, 38, 3087. These methacrylates, can then be subjected to post-polymerization processing. Specifically, hydrolysis (e.g. with trifluoroacetic acid or HCl) will cleave the *tert*-butyl group from the *tert*-butyl methacrylate, leaving a carboxylic acid moiety. This group is highly hydrophilic and allows for additional chemistries to be performed if so desired, for example, allowing one to tune the biodegradation rate of the polymer. With respect to benzyl substituted methacrylates, hydrogenation with activated palladium on carbon with H₂(g) will also yield a carboxylic acid moiety.

To the extent that disulfide bonds may be cleaved to form thiol groups during processing, they may be reformed via oxidation (e.g., with FeCl₃ as described above) in some embodiments.

In other embodiments of the invention, highly biocompatible polymers such as ones containing the following general structure can be created, 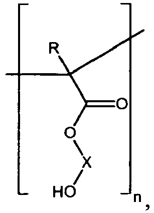 where R and X can be any of a wide range of organic groups, for example, R may be H or C₁-C₅ alkyl and X may be C₁-C₅ alkyl, among many other possibilities. As a specific example, hydroxyethyl methacrylate, where R = H and X = (-CH₂-)₂ can be polymerized via ATRP to produce disulfide containing, biodegradable, biocompatible polymers.

As noted above, due to the nature of ATRP, a terminal halide remains at the end of the polymer after polymerization, which can be further reacted to cap the polymer or which can be used for chain extension, for example, with hydrophilic, hydrophobic, biocompatible, protein resistant or bioactive end capping groups or polymer chains.

As a specific example, amphiphilic polymers may be created by polymerization of a hydrophobic chain followed by polymerization of a hydrophilic chain, or vice versa. For instance, a styrene chain may first be polymerized, followed by polymerization of a hydroxyethyl methacrylate chain or by polymerization of a *t*-butyl methacrylate or benzyl methacrylate chain (followed by conversion to carboxyl groups as discussed above). Amphiphilic polymers are known to form micelles, if present in sufficient concentrations.

Other non limiting examples of polymers in which disulfide linkages may be incorporated to modify degradation in accordance with the invention may be selected from the following among others: polyhydroxy acids and other biodegradable polyesters with internal disulfide linkages (e.g. polylactide, polyglycolide, poly(lactide-*co*-glycolide) and polycaprolactone), polyorthoesters and polysulphones with internal disulfide linkages, polyanhydrides with internal disulfide linkages, polysaccharides, polyamino acids and protein based polymers with internal disulfide linkages, polyurethanes with internal disulfide linkages, polyesters (e.g., polyethylene terephthalate) and polyester amides with internal disulfide linkages, polyvinyl alcohols with internal disulfide linkages, polyvinyl acetates with internal disulfide linkages, polyethers with internal disulfide linkages, polyvinyl aromatics with internal disulfide linkages, silicones and siloxane polymers with internal disulfide linkages, hydrophobic polymers with internal disulfide linkages such as those based on styrenes, butylenes and various dienes, including copolymers thereof, for example, styrene-*b*-isobutylene-*b*-styrene (SIBS), styrene-*b*-isoprene-*b*-styrene (SIS), styrene-*b*-butadiene-*b*-styrene (SBS) copolymers.

Such polymers may be formed, for example, by a polymerization reaction (e.g., condensation reaction, addition reaction, etc.) to provide chain extension from a suitable moiety (e.g., a moiety containing a disulfide bond, such as a disulfide-containing polymer or a disulfide-containing small molecule) or by coupling of a pre-formed polymer to a disulfide-containing moiety. Such polymers may also be formed by coupling thiol terminated polymers together to form higher molecular weight polymers with internal disulfide bridges, among other methods.

Still other non-limiting examples of polymers in which disulfide linkages may be incorporated to modify degradation in accordance with the invention (not necessarily exclusive of those above) may be selected from the following among others: polycarboxylic acid polymers and copolymers including polyacrylic acids; acetal polymers and copolymers; cellulosic polymers and copolymers, including cellulose acetates, cellulose nitrates, cellulose propionates, cellulose acetate butyrates, cellophanes, rayons, rayon triacetates, and cellulose ethers such as carboxymethyl celluloses and hydroxyalkyl celluloses; polyoxymethylene polymers and copolymers; polyimide polymers and copolymers such as polyether block imides and polyether block amides, polyamidimides, polyesterimides, and polyetherimides; polysulfone polymers and copolymers including polyarylsulfones and polyethersulfones; polyamide polymers and copolymers including nylon 6,6, nylon 12, polycaprolactams and polyacrylamides; polycarbonates; polyacrylonitriles; polyvinylpyrrolidones; polymers and copolymers of vinyl monomers including polyvinyl alcohols, polyvinyl halides such as polyvinyl chlorides, ethylene-vinyl acetate copolymers, polyvinylidene chlorides, polyvinyl ethers such as polyvinyl methyl ethers, styrene-maleic anhydride copolymers, vinyl-aromatic-alkylene copolymers, acrylonitrile-styrene copolymers, acrylonitrile-butadiene-styrene copolymers, polyvinyl ketones, polyvinylcarbazoles, and polyvinyl esters such as polyvinyl acetates; polybenzimidazoles; ethylene-methacrylic acid copolymers and ethylene-acrylic acid copolymers, where some of the acid groups can be neutralized with either zinc or sodium ions (commonly known as ionomers); polyalkyl oxide polymers and copolymers including polyethylene oxides (PEO); polyesters including polyethylene terephthalate and aliphatic polyesters such as polymers and copolymers of lactide (which includes lactic acid as well as d-,1- and meso lactide), epsilon-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, and 6,6-dimethyl-1,4-dioxan-2-one (a copolymer of poly(lactic acid) and poly(caprolactone) is one specific example); polyether polymers and copolymers including polyarylethers such as polyphenylene ethers, polyether ketones, polyether ether ketones; polyphenylene sulfides; polyisocyanates; polyolefin polymers and copolymers, including polyalkylenes such as polypropylenes, polyethylenes (low and high density, low and high molecular weight), polybutylenes (such as polybut-1-ene and polyisobutylene), polyolefin elastomers (e.g., santoprene), ethylene propylene diene monomer (EPDM) rubbers, poly-4-methyl-pen-1-enes, ethylene-alpha-olefin copolymers, ethylene-methyl methacrylate copolymers and ethylene-vinyl acetate copolymers; fluorinated polymers and copolymers, including polytetrafluoroethylenes (PTFE), poly(tetrafluoroethylene-co-hexafluoropropene) (FEP), modified ethylene-tetrafluoroethylene copolymers (ETFE), and polyvinylidene fluorides (PVDF); silicone polymers and copolymers; thermoplastic polyurethanes (TPU); elastomers such as elastomeric polyurethanes and polyurethane copolymers (including block and random copolymers that are polyether based, polyester based, polycarbonate based, aliphatic based, aromatic based and mixtures thereof); p-xylylene polymers; polyiminocarbonates; copoly(ether-esters) such as polyethylene oxide-polylactic acid copolymers; polyphosphazines; polyalkylene oxalates; polyoxaamides and polyoxaesters (including those containing amines and/or amido groups); polyorthoesters; biopolymers, such as polypeptides, proteins, and polysaccharides; as well as further copolymers of the above.

In some embodiments, polymers having disulfide linkages may be formed by linking thiol terminated polymers (e.g., polymer chains having thiol moieties at one or both ends), for example, disulfide linkages may be created from thiols by oxidation.

The polymeric regions of the medical devices comprise one or more therapeutic agents, in addition to one or more biodegradable polymers. "Therapeutic agents," "drugs," "pharmaceutically active agents," "pharmaceutically active materials," and other related terms may be used interchangeably herein.

The therapeutic agent(s) is/are covalently coupled along the backbone of the biodegradable polymers or at the termini by a disulfide bond. For example, many therapeutic agents possess thiol groups which would allow covalent linkage into the backbone of the biodegradable polymers. As another example, many therapeutic agents can be modified with sulfide (-S-) or thiol groups, for example, to allow for creation of disulfide linkages by oxidation.

Such therapeutic-agent-containing polymeric matrices may be, for example, injected into a specific body site or applied to a device which would be inserted or implanted at a specific body site. As the matrix degrades, a controllable release of the therapeutic agent may be achieved.

The rate of release of a therapeutic agent from a polymeric region in accordance with the invention will depend on processes such as diffusion and bond degradation, which in turn depends on the nature of the therapeutic agent(s) (e.g., hydrophobic, hydrophilic, amphiphilic, etc.) and the nature of the polymer(s), including the monomer type (hydrophobic, hydrophilic, or mixtures thereof), the amount of and structural location of the disulfide linkages, the polymer architecture, and so forth. By varying such parameters, a large range of drug release and polymer degradation rates can be achieved, yielding a highly tunable drug delivery device.

Exemplary therapeutic agents for use in conjunction with the present invention include the following: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anticoagulants such as D-Phe-Pro-Arg chloromethyl ketone, an RGD peptide-containing compound, heparin, hirudin, antithrombin compounds, platelet receptor antagonists, antithrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, aminoglycosides and nitrofurantoin; (m) cytotoxic agents, cytostatic agents and cell proliferation affectors; (n) vasodilating agents; (o) agents that interfere with endogenous vasoactive mechanisms; (p) inhibitors of leukocyte recruitment, such as monoclonal antibodies; (q) cytokines; (r) hormones; (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin, (t) alpha receptor antagonist (such as doxazosin, Tamsulosin) and beta receptor agonists (such as dobutamine, salmeterol), beta receptor antagonist (such as atenolol, metaprolol, butoxamine), angiotensin-II receptor antagonists (such as losartan, valsartan, irbesartan, candesartan and telmisartan), and antispasmodic drugs (such as oxybutynin chloride, flavoxate, tolterodine, hyoscyamine sulfate, diclomine) (u) bARKct inhibitors, (v) phospholamban inhibitors, (w) Serca 2 gene/protein, (x) immune response modifiers including aminoquizolines, for instance, imidazoquinolines such as resiquimod and imiquimod, (y) human apolioproteins (e.g., AI, AII, AIII, AIV, AV, etc.) (z) selective estrogen receptor modulators (SERMs) such as raloxifene, lasofoxifene, arzoxifene, miproxifene, ospemifene, PKS 3741, MF 101 and SR 16234, (aa) PPAR agonists such as rosiglitazone, pioglitazone, netoglitazone, fenofibrate, bexaotene, metaglidasen, rivoglitazone and tesaglitazar, (bb) prostaglandin E agonists such as alprostadil or ONO 8815Ly, (cc) thrombin receptor activating peptide (TRAP), (dd) vasopeptidase inhibitors including benazepril, fosinopril, lisinopril, quinapril, ramipril, imidapril, delapril, moexipril and spirapril, (ee) thymosin beta 4, (ff) phospholipids including phosphorylcholine, phosphatidylinositol and phosphatidylcholine, and (gg) VLA-4 antagonists and VCAM-1 antagonists.

Numerous therapeutic agents, not necessarily exclusive of those listed above, have been identified as candidates for vascular treatment regimens, for example, as agents targeting restenosis (antirestenotics). Such agents are useful for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists such as bosentan, sitaxsentan sodium, atrasentan, endonentan, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) Angiotensin Converting Enzyme (ACE) inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (1) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, atorvastatin, fluvastatin, simvastatin and cerivastatin, (u) fish oils and omega-3-fatty acids, (v) free-radical scavengers/antioxidants such as probucol, vitamins C and E, ebselen, trans-retinoic acid, SOD (orgotein) and SOD mimics, verteporfin, rostaporfin, AGI 1067, and M 40419, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) matrix metalloprotease (MMP) pathway inhibitors such as marimastat, ilomastat, metastat, pentosan polysulfate, rebimastat, incyclinide, apratastat, PG 116800, RO 1130830 or ABT 518, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate , nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), olimus family drugs (e.g., sirolimus, everolimus, tacrolimus, zotarolimus, etc.), cerivastatin, flavopiridol and suramin, (aa) matrix deposition/organization pathway inhibitors such as halofuginone or other quinazolinone derivatives pirfenidone and tranilast, (bb) endothelialization facilitators such as VEGF and RGD peptide, (cc) blood rheology modulators such as pentoxifylline and (dd) glucose cross-link breakers such as alagebrium chloride (ALT-711).

Numerous antineoplastic/antiproliferative/anti-mitotic agents, not necessarily exclusive of those listed above, many of which have been identified as having antineoplastic effects, are useful for the practice of the present invention and include one or more of the following: antimetabolites such as folic acid analogs/antagonists (e.g., methotrexate, etc.), purine analogs (e.g., 6-mercaptopurine, thioguanine, cladribine, which is a chlorinated purine nucleoside analog, etc.) and pyrimidine analogs (e.g., cytarabine, fluorouracil, etc.), alkaloids including taxanes (e.g., paclitaxel, docetaxel, etc.), alkylating agents such as alkyl sulfonates, nitrogen mustards (e.g., cyclophosphamide, ifosfamide, etc.), nitrosoureas, ethylenimines and methylmelamines, other aklyating agents (e.g., dacarbazine, etc.), antibiotics and analogs (e.g., daunorubicin, doxorubicin, idarubicin, mitomycin, bleomycins, plicamycin, etc.), platinum complexes (e.g., cisplatin, carboplatin, etc.), antineoplastic enzymes (e.g., asparaginase, etc.), agents affecting microtubule dynamics (e.g., vinblastine, vincristine, colchicine, Epo D, epothilone), caspase activators, proteasome inhibitors, angiogenesis inhibitors (e.g., statins such as endostatin, cerivastatin and angiostatin, squalamine, etc.), olimus family drugs, etoposides, as well as many others (e.g., hydroxyurea, flavopiridol, procarbizine, mitoxantrone, campothecin, etc.), and combinations of the foregoing, among other known antineoplastic/antiproliferative/anti-mitotic agents.

Where present, a wide range of therapeutic agent loadings may be used in conjunction with the medical devices of the present invention. Typical loadings range, for example, from than 1 wt% or less to 2 wt% to 5 wt% to 10 wt% to 25 wt% or more of the polymeric region.

Numerous techniques are available for forming polymeric regions in accordance with the present invention.

For example, where a polymeric region is formed from one or more polymers having thermoplastic characteristics, a variety of standard thermoplastic processing techniques may be used to form the polymeric region. Using these techniques, a polymeric region can be formed, for instance, by (a) first providing a melt that contains polymer(s) and any optional agents such as therapeutic agent(s) and (b) subsequently cooling the melt. Examples of thermoplastic processing techniques, including compression molding, injection molding, blow molding, spraying, vacuum forming and calendaring, extrusion into sheets, fibers, rods, tubes and other cross-sectional profiles of various lengths, and combinations of these processes. Using these and other thermoplastic processing techniques, entire devices or portions thereof can be made.

Other processing techniques besides thermoplastic processing techniques may also be used to form the polymeric regions of the present invention, including solvent-based techniques. Using these techniques, a polymeric region can be formed, for instance, by (a) first providing a solution or dispersion that contains polymer(s) and any optional agents such as therapeutic agent(s), and (b) subsequently removing the solvent. The solvent that is ultimately selected will contain one or more solvent species, which are generally selected based on their ability to dissolve at least one of the polymer(s) that form the polymeric region, in addition to other factors, including drying rate, surface tension, etc. In certain embodiments, the solvent is selected based on its ability to dissolve or disperse any optional agents such as therapeutic agent(s) as well. Preferred solvent-based techniques include, but are not limited to, solvent casting techniques, spin coating techniques, web coating techniques, solvent spraying techniques, dip coating techniques, techniques involving coating via mechanical suspension including air suspension, ink jet techniques, solvent spinning, electrostatic techniques, and combinations of these processes.

In some embodiments of the invention, a polymer containing solution (where solvent-based processing is employed) or a polymer melt (where thermoplastic processing is employed) is applied to a substrate to form a polymeric region. For example, the substrate can correspond to all or a portion of an implantable or insertable medical device to which a polymeric coating is applied, for example, by spraying, dipping, extrusion, and so forth. The substrate can also be, for example, a template, such as a mold, from which the polymeric region is removed after solidification. In other embodiments, for example, extrusion and co-extrusion techniques, one or more polymeric regions are formed without the aid of a substrate. In one specific example, an entire medical device is extruded. In another, a polymeric coating layer is co-extruded along with and underlying medical device body.

### EXAMPLES

### Example 1 (Reference)

A biodegradable biocompatible system based on poly(hydroxyethyl methacrylate) segments linked by disulfide bonds may be formed and applied to a coronary stent, along with paclitaxel and a suitable solvent, via a spray coating process.

### Example 2 (Reference)

An ABA triblock copolymer with a poly(n-butyl acrylate) midblock of ∼40,000 daltons formed from poly(n-butyl acrylate) with MW of ∼10,000 daltons and a PDI of ∼1.1 between disulfide linkages and with polystyrene endblocks of ∼10,000 to ∼15,000 daltons formed from polystyrene with MW -2500 daltons and a PDI ∼1.1 between disulfide linkages, is spray coated onto a coronary stent, along with from 0.1 to 50% by weight of paclitaxel from a suitable solvent.

### Example 3 (Reference)

An ABA triblock copolymer with a poly(n-butyl acrylate) midblock of ∼40,000 daltons formed from poly(n-butyl acrylate) with MW of ∼10,000 daltons and a PDI of ∼1.1 between disulfide linkages and with poly(methyl methacrylate) endblocks of ∼10,000 to ∼15,000 formed from poly(methyl methacrylate) with MW ∼2500 daltons and a PDI ∼1.1 between disulfide linkages, is spray coated onto a coronary stent, along with from 0.1 to 50% paclitaxel.

### Example 4 (Reference)

The polymer/drug combination from Example 3 is roll coated onto the stent, rather than spray coated.

### Example 5 (Reference)

The polymer drug combination from Example 3 is dip coated onto the stent, rather than spray coated.

## Claims

1. An implantable or insertable medical device comprising a non-crosslinked biodegradable polymeric region, said non-crosslinked biodegradable polymeric region comprising a polymer that comprises a plurality of first polymer segments linked by one or more disulfide linkages, wherein said polymeric region comprises a therapeutic agent that is covalently linked to the polymer by a disulfide bond.

2. The implantable or insertable medical device of claim 1, wherein the polymer is a star polymer that comprises a core with a plurality of disulfide linkages and wherein the first segments extend out from the core, and
wherein the core comprises a polymer of a dimethacrylate compound that comprises a disulfide linkage which separates two methacrylate groups.

3. The implantable or insertable medical device of claim 1, wherein the first polymer segments have a polydispersity of less than 1.2.

4. The implantable or insertable medical device of claim 1, wherein the first polymer segments are hydrophilic segments, or specifically hydrophilic segments selected from hydroxyalkyl (meth)acrylate segments and (meth)acrylic acid segments.

5. The implantable or insertable medical device of claim 1, wherein the first polymer segments are hydrophobic segments, or specifically hydrophobic segments selected from styrene, acrylic esters, and methacrylic esters.

6. The implantable or insertable medical device of claim 1, wherein the first polymer segments are amphiphilic segments, or wherein the first polymer segments are low Tg segments, or wherein the first polymer segments are high Tg segments.

7. The implantable or insertable medical device of claim 1, wherein the polymer further comprises a plurality of second segments linked by one or more disulfide linkages, said second segments differing from the first segments in monomer content.

8. The implantable or insertable medical device of claim 7, wherein the first segments are hydrophilic and the second segments are hydrophobic, or wherein the first segments are high Tg segments and the second segments are low Tg segments.

9. The implantable or insertable medical device of claim 1 or 7, wherein the polymer is a linear polymer, or wherein the polymer is a branched polymer.

10. The implantable or insertable medical device of claim 1 or 7, wherein the polymer is a star polymer that comprises a core with a plurality of disulfide linkages and wherein the first and second segments extend out from the core.

11. The implantable or insertable medical-device of claim 7, wherein the first and second segments each comprises monomers selected from acrylic monomers, methacrylic monomers, styrene, substituted styrene monomers, vinyl pyridine monomers, substituted vinyl pyridine monomers, acrylamide monomers, methacrylamide monomers.

12. The implantable or insertable medical device of claim 7, wherein the polymer further comprises a plurality of third segments linked by one or more disulfide linkages, said third segments differing from the first and second segments in monomer content.

13. The implantable or insertable medical device of claim 1, wherein said polymeric region corresponds to an entire medical device or to an entire component of a medical device.

14. The implantable or insertable medical device of claim 1, wherein said polymeric region is in the form of a layer that at least partially covers an underlying substrate.

15. The implantable or insertable medical device of claim 1, wherein said therapeutic agent is selected from antiproliferative agents, antithrombotic agents, endothelial cell growth promoters, antimicrobial agents, analgesic agents, antirestenotic agents, and anti-inflammatory agents.

16. The implantable or insertable medical device of claim 1, wherein the medical device is a blood contacting medical device.

17. The medical device of claim 1, wherein said medical device is a stent.

18. The device of claim 1, wherein said biodegradable first polymer segments are selected from polyesters, polyanhydrides, polyesteramides, and polysaccharides.

## Patentansprüche

1. Eine implantierbare oder einführbare medizinische Vorrichtung, umfassend eine nicht vernetzte, biologisch abbaubare polymere Region, wobei die nicht vernetzte, biologisch abbaubare polymere Region ein Polymer umfasst, welches eine Vielzahl an ersten Polymersegmenten umfasst, die durch eine oder mehrere Disulfid-Bindungen verbunden sind, wobei die polymere Region einen therapeutischen Wirkstoff umfasst, der kovalent mit dem Polymer durch eine Disulfidbindung verbunden ist.

2. Die implantierbare oder einführbare medizinische Vorrichtung gemäß Anspruch 1, wobei das Polymer ein Sternpolymer ist, welches einen Kern mit einer Vielzahl von Disulfid-Bindungen umfasst, und wobei sich die ersten Segmente vom Kern weg erstrecken, und wobei der Kern ein Polymer einer Dimethacrylatverbindung umfasst, die eine DisulfidBindung umfasst, welche zwei Methacrylatgruppen trennt.

3. Die implantierbare oder einführbare medizinische Vorrichtung gemäß Anspruch 1, wobei die ersten Polymersegmente eine Polydispersität von weniger als 1,2 aufweisen.

4. Die implantierbare oder einführbare medizinische Vorrichtung gemäß Anspruch 1, wobei die ersten Polymersegmente hydrophile Segmente sind, oder spezifisch hydrophile Segmente, ausgewählt aus Hydroxyalkyl(meth)acrylatsegmenten und (Meth)acrylsäuresegmenten sind.

5. Die implantierbare oder einführbare medizinische Vorrichtung gemäß Anspruch 1, wobei die ersten Polymersegmente hydrophobe Segmente sind, oder spezifisch hydrophobe Segmente, ausgewählt aus Styrol, Acrylsäureestern und Methacrylsäureestern sind.

6. Die implantierbare oder einführbare medizinische Vorrichtung gemäß Anspruch 1, wobei die ersten Polymersegmente amphiphile Segmente sind, oder wobei die ersten Polymersegmente Segmente mit niedrigem Tg sind, oder wobei die ersten Polymersegmente Segmente mit hohem Tg sind.

7. Die implantierbare oder einführbare medizinische Vorrichtung gemäß Anspruch 1, wobei das Polymer ferner eine Vielzahl an zweiten Segmenten umfasst, die durch eine oder mehrere Disulfid-Bindungen verbunden sind, wobei die zweiten Segmente sich von den ersten Segmenten in ihrem Monomergehalt unterscheiden.

8. Die implantierbare oder einführbare medizinische Vorrichtung gemäß Anspruch 7, wobei die ersten Segmente hydrophil sind und die zweiten Segmente hydrophob sind, oder wobei die ersten Segmente Segmente mit hohem Tg sind und die zweiten Segmente Segmente mit niedrigem Tg sind.

9. Die implantierbare oder einführbare medizinische Vorrichtung gemäß Anspruch 1 oder 7, wobei das Polymer ein lineares Polymer ist oder wobei das Polymer ein verzweigtes Polymer ist.

10. Die implantierbare oder einführbare medizinische Vorrichtung gemäß Anspruch 1 oder 7, wobei das Polymer ein Stempolymer ist, welches einen Kern mit einer Vielzahl an Disulfid-Bindungen umfasst und wobei sich die ersten und zweiten Segmente vom Kern weg erstrecken.

11. Die implantierbare oder einführbare medizinische Vorrichtung gemäß Anspruch 7, wobei die ersten und zweiten Segmente jeweils Monomere umfassen, die ausgewählt sind aus acrylischen Monomeren, methacrylischen Monomeren, Styrol, substituierten StyrolMonomeren, Vinylpyridin-Monomeren, substituierten Vinylpyridin-Monomeren, Acrylamid-Monomeren, Methacrylamid-Monomeren.

12. Die implantierbare oder einführbare medizinische Vorrichtung gemäß Anspruch 7, wobei das Polymer ferner eine Vielzahl an dritten Segmenten umfasst, die durch eine oder mehrere Disulfid-Bindungen verbunden sind, wobei sich die dritten Segmente von den ersten und zweiten Segmenten in ihrem Monomergehalt unterscheiden.

13. Die implantierbare oder einführbare medizinische Vorrichtung gemäß Anspruch 1, wobei die polymere Region einer ganzen medizinischen Vorrichtung oder einem ganzen Bestandteil einer medizinischen Vorrichtung entspricht.

14. Die implantierbare oder einführbare medizinische Vorrichtung gemäß Anspruch 1, wobei die polymere Region in der Form einer Schicht, die mindestens teilweise ein darunterliegendes Substrat bedeckt, vorliegt.

15. Die implantierbare oder einführbare medizinische Vorrichtung gemäß Anspruch 1, wobei der therapeutische Wirkstoff ausgewählt ist aus antiproliferativen Wirkstoffen, antithrombotischen Wirkstoffen, Endothelzellen-Wachstumsförderern, antimikrobiellen Wirkstoffen, schmerzstillenden Wirkstoffen, antirestenotischen Wirkstoffen und entzündungshemmenden Wirkstoffen.

16. Die implantierbare oder einführbare medizinische Vorrichtung gemäß Anspruch 1, wobei die medizinische Vorrichtung eine mit Blut in Berührung kommende medizinische Vorrichtung ist.

17. Die medizinische Vorrichtung gemäß Anspruch 1, wobei die medizinische Vorrichtung ein Stent ist.

18. Die Vorrichtung gemäß Anspruch 1, wobei die biologisch abbaubaren ersten Polymersegmente ausgewählt sind aus Polyestern, Polyanhydriden, Polyesteramiden und Polysacchariden.

## Revendications

1. Dispositif médical implantable ou insérable comprenant une région polymère biodégradable non réticulée, ladite région polymère biodégradable non réticulée comprenant un polymère qui comprend une pluralité de premiers segments de polymère liés par une ou plusieurs liaisons disulfure, où ladite région polymère comprend un agent thérapeutique qui est lié par covalence au polymère par une liaison disulfure.

2. Dispositif médical implantable ou insérable selon la revendication 1, où le polymère est un polymère en étoile qui comprend un coeur avec une pluralité de liaisons disulfure et où les premiers segments s'étendent depuis le coeur, et
où le coeur comprend un polymère d'un composé diméthacrylate qui comprend une liaison disulfure qui sépare deux groupes méthacrylate.

3. Dispositif médical implantable ou insérable selon la revendication 1, où les premiers segments de polymère ont une polydispersité inférieure à 1,2.

4. Dispositif médical implantable ou insérable selon la revendication 1, où les premiers segments de polymère sont des segments hydrophiles, ou spécifiquement des segments hydrophiles choisis parmi des segments de (méth)acrylate d'hydroxyalkyle et des segments d'acide (méth)acrylique.

5. Dispositif médical implantable ou insérable selon la revendication 1, où les premiers segments de polymère sont des segments hydrophobes, ou spécifiquement des segments hydrophobes choisis parmi le styrène, les esters acryliques et les esters méthacryliques.

6. Dispositif médical implantable ou insérable selon la revendication 1, où les premiers segments de polymère sont des segments amphiphiles, ou bien où les premiers segments de polymère sont des segments à basse Tg, ou bien où les premiers segments de polymère sont des segments à haute Tg.

7. Dispositif médical implantable ou insérable selon la revendication 1, où le polymère comprend en outre une pluralité de seconds segments liés par une ou plusieurs liaisons disulfure, lesdits seconds segments différant des premiers segments dans la teneur en monomères.

8. Dispositif médical implantable ou insérable selon la revendication 7, où les premiers segments sont hydrophiles et les seconds segments sont hydrophobes, ou bien où les premiers segments sont des segments à haute Tg et les seconds segments sont des segments à basse Tg.

9. Dispositif médical implantable ou insérable selon la revendication 1 ou 7, où le polymère est un polymère linéaire ou bien où le polymère est un polymère ramifié.

10. Dispositif médical implantable ou insérable selon la revendication 1 ou 7, où le polymère est un polymère en étoile qui comprend un coeur avec une pluralité de liaisons disulfure et où les premiers et seconds segments s'étendent depuis le coeur.

11. Dispositif médical implantable ou insérable selon la revendication 7, où les premiers et seconds segments comprennent chacun des monomères choisis parmi les monomères acryliques, les monomères méthacryliques, le styrène, les monomères de styrène substitués, les monomères de vinylpyridine, les monomères de vinylpyridine substitués, les monomères d'acrylamide, les monomères de méthacrylamide.

12. Dispositif médical implantable ou insérable selon la revendication 7, où le polymère comprend en outre une pluralité de troisièmes segments liés par une ou plusieurs liaisons disulfure, lesdits troisièmes segments différant des premiers et seconds segments dans la teneur en monomères.

13. Dispositif médical implantable ou insérable selon la revendication 1, où ladite région polymère correspond à un dispositif médical entier ou à un composé entier d'un dispositif médical.

14. Dispositif médical implantable ou insérable selon la revendication 1, où ladite région polymère est sous forme d'une couche qui couvre au moins partiellement un substrat sous-jacent.

15. Dispositif médical implantable ou insérable selon la revendication 1, où ledit agent thérapeutique est choisi parmi les agents antiprolifératifs, les agents antithrombotiques, les promoteurs de la croissance des cellules endothéliales, les agents antimicrobiens, les agents analgésiques, les agents antiresténose et les agents anti-inflammatoires.

16. Dispositif médical implantable ou insérable selon la revendication 1, où le dispositif médical est un dispositif médical entrant en contact avec le sang.

17. Dispositif médical selon la revendication 1, où ledit dispositif médical est un stent.

18. Dispositif selon la revendication 1, où lesdits premiers segments de polymère biodégradables sont choisis parmi les polyesters, les polyanhydrides, les polyestéramides et les polysaccharides.
